(19) Europäisches Patentamt
European Patent Office
Office européen des brevets

(11) Veröffentlichungsnummer: **0 050 779**
**B1**

(12) # EUROPÄISCHE PATENTSCHRIFT

(45) Veröffentlichungstag der Patentschrift: **16.05.84**

(51) Int. Cl.[3]: **C 07 C 103/34**, C 07 C 103/44, C 07 C 51/60, C 07 D 241/44

(21) Anmeldenummer: **81108194.2**

(22) Anmeldetag: **12.10.81**

(54) **N-alkylierte Formamide, Verfahren zu ihrer Herstellung und ihre Verwendung.**

(30) Priorität: **23.10.80 DE 3039999**

(43) Veröffentlichungstag der Anmeldung: **05.05.82 Patentblatt 82/18**

(45) Bekanntmachung des Hinweises auf die Patenterteilung: **16.05.84 Patentblatt 84/20**

(84) Benannte Vertragsstaaten: **BE CH DE FR GB IT LI**

(56) Entgegenhaltungen:
**DE - A - 2 053 840**
**DE - A - 2 425 448**
**DE - A - 2 523 633**
**US - A - 3 894 958**

**CHEMICAL ABSTRACTS, Band 89, Nr. 21, 20. November 1978, Seite 542, Nr. 179214k Columbus, Ohio, U.S.A. B. DEDERICHS et al.: "Radiation chemical addition of dimethylformamide to alpha-olefins"**
**JOURNAL OF THE AMERICAN CHEMICAL SOCIETY, Band 99, Nr. 6, 16. März 1977, Seiten 1831-1835 U.S.A. G.M. McNAMEE et al.: "Electrochemical reduction of 1-iododecane and 1-bromodecane at a mercury cathode in dimethylformamide"**

(73) Patentinhaber: **BAYER AG, Konzernverwaltung RP Patentabteilung, D-5090 Leverkusen 1 Bayerwerk (DE)**

(72) Erfinder: **Blank, Heinz Ulrich, Dr., Am Geusfelde 35, D-5068 Odenthal (DE)**
Erfinder: **Langenfeld, Norbert, Dr., Hofstrasse 53, D-5000 Köln 80 (DE)**
Erfinder: **Heydkamp, Wolfgang, Dr., Gruener Weg 18, D-5090 Leverkusen (DE)**

**EP 0 050 779 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# N-alkylierte Formamide, Verfahren zu ihrer Herstellung und ihre Verwendung

Die vorliegende Erfindung betrifft neue N-alkylierte Formamide, ein Verfahren zu ihrer Herstellung und ihre Verwendung als Beschleuniger beim Austausch von Hydroxylgruppen in organischen Verbindungen gegen Chlor oder Brom.

Aus C.A. 89, 179214k (1978) ist es bekannt, durch strahlenchemische Addition von Dimethylformamid an $\alpha$-Olefine, wie 1-Hexen, 1-Octen und 1-Decen, ein Gemisch der entsprechenden N,N-Dimethyl-alkanamide und N-Methyl-N-alkylformamide zu erhalten.

Aus J. Am. Chem. Soc. 99, 1831–1835 (1977) ist es bekannt, durch Elektrolyse von Brom- oder Joddecan in Gegenwart von N-Methylformamid N-Methyl-N-decyl-formamid zu erhalten.

In DE-OS 20 53 840 wird die Herstellung von Carbamidsäurechloriden durch Umsetzung von N,N-disubstituierten Formamiden mit Phosphortrihalogeniden und Thionylhalogeniden beschrieben, wobei die Substituenten am Stickstoff $C_1$—$C_8$-Alkyl sein können.

US 3.894.958 beschreibt synthetische Schmieröle aus Gemischen von N,N-disubstituierten Carbonsäureamiden, bei denen einer der Substituenten am Stickstoff ein sekundäres Alkylradikal mit 5—25 C-Atomen ist.

Aus DE-OS 24 25 448 sind Katalysatoren für Polyurethanschäume bekannt, die die allgemeine Formel

X—NAlkyl—A—NAlkyl—A—NAlkyl—X

haben, wobei

A $C_1$—$C_6$-Alkylengruppen und
X Acyl bedeuten.

Die Acylgruppen werden durch Säureanhydride oder -chloride eingeführt, was die Einführung der Formylgruppe ausschließt. Die DE-OS 25 23 633 beschreibt Alkylendiamine oder Dialkylentriamine, bei denen ein N-Atom eine Acylgruppe trägt und alle weiteren H-Atome an den N-Atomen durch $C_1$—$C_5$-Alkylgruppen substituiert sind, als Katalysatoren für die Herstellung von Polyurethan-Kunststoffen.

Es wurden die neuen N-alkylierten Formamide der Formel

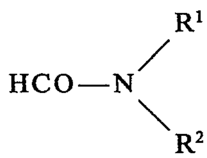

(I)

gefunden, in der

$R^1$ niederes Alkyl und
$R^2$ geradkettiges Alkyl mit 14—30 C-Atomen oder die Gruppe

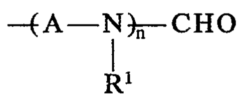

bedeutet, wobei

A geradkettiges oder verzweigtes Alkandiyl mit 2 bis 8 C-Atomen,
$R^1$ niederes Alkyl und
n eine ganze Zahl von 2 bis 6

bedeutet.

Die Alkylreste $R^1$ bedeuten niederes Alkyl, beispielsweise mit 1 bis 4 C-Atomen. Solche Reste können geradkettig oder verzweigt, bevorzugt geradkettig, sein. Beispiele hierfür sind: Methyl, Ethyl, Propyl, Isopropyl, Butyl, Isobutyl, bevorzugt Methyl.

Die Alkylreste $R^2$ haben erfindungsgemäß 14—30, bevorzugt 16 bis 22 C-Atome und sind geradkettig. Beispiele hierfür sind: Myristyl, Palmityl, Heptadecanyl, Stearyl, Nonadecanyl, Eicosanyl, Docosanyl und Triacontanyl.

Für den Fall, daß $R^2$ die Gruppe

$$-(A-NR^1)_n-CHO$$

bedeutet, hat $R^1$ den genannten Bedeutungsumfang und n die Bedeutung einer ganzen Zahl von 2 bis 6, bevorzugt 2 bis 4, besonders bevorzugt 2 bis 3, während A geradkettiges oder verzweigtes Alkandiyl mit 2 bis 8, bevorzugt 2 bis 4, besonders bevorzugt 2 C-Atomen, darstellt, wie

Ethylen, Propylen-(1,2), Propylen-(1,3), Butylen-1,2), Butylen-(1,3), Butylen-(1,4), Hexandiyl oder Octandiyl.

Beispiele für solche Gruppen sind:

3,6-Dimethyl-6-formyl-3,6-diaza-hexyl,
3,6-Diethyl-6-formyl-3,6-diaza-hexyl,
3,6-Dibutyl-6-formyl-3,6-diaza-hexyl,
3,6,9-Trimethyl-9-formyl-3,6,9-triaza-nonyl,
3,6,9,12,15,18-Hexamethyl-18-formyl-hexaaza-octadecyl,
2,3,5,6-Tetramethyl-6-formyl-3,6-diaza-hexyl,
4,8-Dimethyl-8-formyl-4,8-diaza-octyl.

Als Beispiele für die erfindnungsgemäßen Verbindungen seien genannt:

N-Myristyl-, N-Palmityl-, N-Heptadecanyl-, N-Stearyl-, N-Nonadecanyl-, N-Eicosanyl-, N-Docosanyl- und N-Triakontanyl-methylformamid, -ethylformamid, -propylformamid und -butylformamid, sowie
$\alpha$, $\omega$-Bisformyl-N,N',N''-trimethyl-diethylentriamin,
$\alpha$, $\omega$-Bisformyl-N,N',N''N'''-tetramethyl-triethylentetramin und
$\alpha$, $\omega$-Bisformyl-N,N',N''N''',$N^{IV}$,$N^{V}$,$N^{VI}$-heptamethy-hexaethylen-heptamin, sowie
$\alpha$, $\omega$-Bisformyl-N,N',N''-trimethyl-dipropylen-(1,2)-triamin.

Bevorzugte Verbindungen im Rahmen der Formel (I) sind solche der Formel

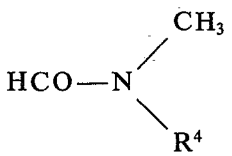

(II)

in der

$R^4$ $C_{16}$—$C_{22}$-Alkyl bedeutet.

Weitere bevorzugte Verbindungen im Rahmen der Formel (I) sind solche der Formel

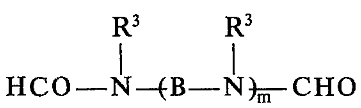

(III)

in der

$R^3$ $C_1$—$C_4$-Alkyl,
B geradkettiges oder verzweigtes $C_2$—$C_4$-Alkandiyl und
m eine ganze Zahl von 2 bis 4

bedeuten.

Weitere besonders bevorzugte Verbindungen im Rahmen der Formel (I) sind solche der Formel

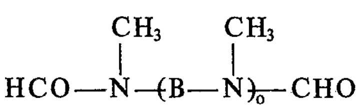

(IV)

in der

o die Zahl 2 oder 3 bedeutet und
B die genannte Bedeutung hat.

Ganz besonders bevorzugt im Rahmen der Formel (IV) sind Verbindungen der Formel

$$HCO-NCH_3-(CH_2-CH_2-NCH_3)_o-CHO \qquad (V)$$

in der

o die angegebene Bedeutung hat.

Die Erfindung betrifft weiterhin Gemische von Stoffen der Formel (I), insbesondere Gemische von Stoffen der Formel (III) beziehungsweise der Formel (IV). Diese Gemische können aus 2 oder mehr Stoffen der genannten Formeln bestehen, bevorzugt aus 2 bis 6 Stoffen, besonders bevorzugt aus 2 bis 4 Stoffen.

Weiterhin wurde ein Verfahren zur Herstellung der N-alkylierten Formamide der Formel

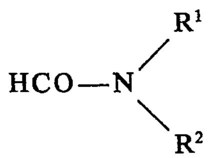

(I)

in der

$R^1$ niederes Alkyl und
$R^2$ geradkettiges Alkyl mit 14—30 C-Atomen oder die Gruppe

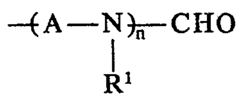

bedeutet, wobei

A geradkettiges oder verzweigtes Alkandiyl,
$R^1$ niederes Alkyl und
n eine ganze Zahl von 2 bis 6

bedeutet,
gefunden, das dadurch gekennzeichnet ist, daß man N-alkylierte Amine der Formel

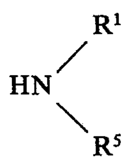

(VI)

in der

$R^1$ die genannte Bedeutung hat und
$R^5$ geradkettiges Alkyl mit 14—30 C-Atomen oder die Gruppe

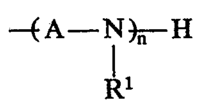

bedeutet, wobei

A geradkettiges oder verzweigtes Alkandiyl
$R^1$ niederes Alkyl und
n eine ganze Zahl von 2 bis 6

bedeutet,
mit Ameisensäure bei erhöhter Temperatur, gegebenenfalls in Gegenwart eines interten Lösungsmittels, umsetzt.

Die Substituenten der erfindungsgemäß einsetzbaren N-alkylierten Amine entsprechen dem oben genannten Bedeutungsumfang. Beispiele für solche N-alkylierte Amine sind:

N-Myristyl-, N-Palmityl-, N-Heptadecanyl-, N-Stearyl-, N-Nonadecanyl-,
N-Eicosanyl-, N-Docosanyl- und
N-Triacontanyl-methylamin, -ethylamin, -propylamin und -butylamin, sowie
N,N',N"-Trimethyl-diethylen-triamin,
N,N',N"-Triethyl-diethylentriamin und
N,N',N"N'",$N^{4'}$,$N^{5'}$,$N^{6'}$-Heptamethyl-hexaethylen-heptamin, sowie
N,N',N"-Trimethyl-dipropylen-(1,2)-triamin.

Bevorzugt werden im erfindungsgemäßen Verfahren N-alkylierte Amine der Formel

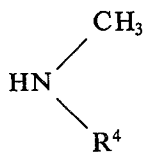

(VII)

eingesetzt, in denen

$R^4$ den oben gegebenen Bedeutungsumfang hat.

Weitere bevorzugt im erfindungsgemäßen Verfahren einsetzbare N-alkylierten Amine sind solche der Formel

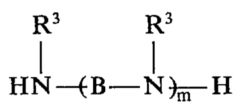

(VIII)

weitere besonders bevorzugt einsetzbare solche der Formel

$$HNCH_3-(B-NCH_3)_o-H \quad (IX)$$

und im Rahmen der Formel (VIII) ganz besonders bevorzugt solche der Formel

$$HNCH_3-(CH_2-CH_2-NCH_3)_o-H \quad (X)$$

worin $R^3$, B, m und o die oben genannte Bedeutung haben.

N-alkylierte Amine der Formeln (VI) bis (X) sind dem Fachmann bekannt. Für den Fall, daß $R^5$ und das davon abgeleitete $R^4$ geradkettige Alkyl mit 14—30 bzw. 16—22 C-Atomen bedeuten, können solche Amine beispielsweise durch Umsetzung von geradkettigen $\alpha$-Halogenparaffinen oder primären Alkoholen mit Mononiederalkylaminen erhalten werden (Houben-Weyl, Methoden der organischen Chemie, Band XI/1, Seite 40, 120, Georg Thieme Verlag 1957). Für den Fall, daß $R^5$ die Gruppe

$$-(A-NR^1)_n-H$$

darstellt, sowie für den Fall der abgeleiteten Formeln (VIII), (IX) und (X) können solche Polyamine durch Umsetzung von Dihalogenalkanen, deren C-Kette geradkettig oder verzweigt sein kann, mit Mononiederalkylaminen erhalten werden (Chem. Ber. 94, 831—833 [1961]). Für den Fall, daß erfindungsgemäß Gemische von Stoffen der Formeln (I), (III), (IV) beziehungsweise (V) erhalten werden sollen, können selbstverständlich Gemische der genannten Amine und/oder Polyamine der Formeln (VI), (VIII), (IX) beziehungsweise (X) im erfindungsgemäßen Verfahren eingesetzt werden. Dies ist dann bevorzugt, wenn bei der technischen Darstellung der einzusetzenden Amine beispielsweise von einem Gemisch verschieden langer geradkettiger $\alpha$-Halogenparaffine ausgegangen wird oder beispielsweise die Umsetzung von Dihalogenalkanen mit Alkylaminen zu einem Gemisch der Polyamine mit verschiedener Anzahl von N-Atomen führt und ei so erhaltenen technischen Gemische aus Kostengründen nicht in die Einzelkomponenten getrennt werden.

Erfindungsgemäß werden das N-alkylierte Amin und die Ameisensäure im Molverhältnis 1:1 bis 20, bevorzugt 1:1 bis 5, besonders bevorzugt 1:1 bis 1,5, bezogen auf jedes acylierbare N-Atom, eingesetzt.

Als Reaktionstemperatur sei beispielsweise 50 bis 150°C genannt. Bevorzugt wird die Reaktion des erfindungsgemäßen Verfahrens bei der Siedetemperatur des Reaktionsgemisches durchgeführt.

Die Reaktion kann bei Normaldruck, erhöhtem oder erniedrigtem Druck durchgeführt werden. Vorzugsweise wird bei Normaldruck oder bei erniedrigtem Druck, beispielsweise im Bereich von 1 bis 1000 mbar, vorzugsweise 50—1000 mbar, gearbeitet.

Zur Verbesserung der Ausbeute durch weitere Vervollständigung der Reaktion des erfindungsgemäßen Verfahrens kann das bei der Reaktion entstehende Wasser während der Reaktion entfernt

werden. Dies kann beispielsweise durch Anwendung erhöhter Temperatur und/oder erniedrigtem Druck und Abdestillieren des Reaktionswassers, durch Wasser entziehende Mittel oder durch Herausschleppen des Reaktionswassers aus dem Reaktionsgemisch durch Azotropdestillation unter Verwendung eines geeigneten Schleppmittels erfolgen. Eine bevorzugte Variante ist die der Wasserentfernung durch azeotrope Destillation. Für diesen Fall wird die erfindungsgemäße Reaktion in Gegenwart eines Lösungsmittels durchgeführt, das mit den Reaktionspartnern unter den Reaktionsbedingungen keine Nebenreaktionen eingeht und das mit Wasser nicht oder nur begrenzt mischbar ist, jedoch mit Wasser ein Azeotrop bildet. Hierfür seien aliphatische oder aromatische Kohlenwasserstoffe, wie Benzol, Toluol, Ethylbenzol, Isopropylbenzol, o- m- und p-Xylol oder Hexan, sowie aliphatische oder aromatische Halogenkohlenstoffwasserstoffe, wie Chlorbenzol, o-, m- und p-Dichlorbenzol oder Dichlorethan, genannt. Bevorzugtes, mit Wasser ein Azeotrop bildendes Lösungsmittel für das erfindungsgemäße Verfahren ist Toluol.

Selbstverständlich können auch andere inerte Lösungsmittel, die mit Wasser kein Azeotrop bilden, im erfindungsgemäßen Verfahren eingesetzt werden.

Alle genannten, ein Azeotrop bildenden oder kein Azeotrop bildenden Lösungsmittel können sowohl einzeln als auch im Gemisch eingesetzt werden. Bevorzugt ist die Verwendung nur eines, ein Azeotrop bildenden Lösungsmittels, besonders bevorzugt Toluol.

Eine weitere bevorzugte Variante ist die Umsetzung des Amins mit Ameisensäure in Abwesenheit eines Lösungsmittels, wobei das entstehende Reaktionswasser durch erhöhte Temperatur, gegebenenfalls bei vermindertem Druck, entfernt wird.

Die Isolierung des Reaktionsproduktes kann beispielsweise durch Abdestillieren des gegebenenfalls verwendeten Lösungsmittels und gegebenenfalls überschüssiger Reaktionspartner erfolgen. Das so erhaltene Produkt ist in den meisten Fällen von genügender Reinheit für die weitere Verwendung. Ist ein weiter gereinigtes Produkt erwünscht, kann die Reinigung nach bekannten Verfahren erfolgen, beispielsweise durch Destillation, Extraktion, Umkristallisation oder Chromatographie.

Selbstverständlich können zur Herstellung der N-alkylierten Formamide der Formel (I) auch andere Herstellungsmethoden für formylierte Amine herangezogen werden. Solche Methoden sind beispielsweise in Houben-Weyl, Methoden der organischen Chemie, 4. Auflage 1958, Band XI/2, Seiten 27 bis 30, beschrieben und umfassen beispielsweise die Umsetzung von N-alkylierten Aminen der Formel (IV) mit Ammoniumformiat, einem Gemisch aus Ameisensäure und Essigsäure, Ameisensäurealkylestern, Formamid, Formylfluorid, Chloral oder Kohlenmonoxid.

Die erfindungsgemäßen N-alkylierten Formamide sind als Beschleuniger beim Austausch von Hydroxylgruppen in organischen Verbindungen gegen Chlor oder Brom verwendbar. Sie sind weiterhin verwendbar als Beschleuniger bei der Umsetzung von phenolischen Verbindungen mit Phosgen zu Arylchlorameisensäureestern.

Bisher wurden als Katalysatoren für solche Reaktionen Dialkylformamide, deren Alkylreste höchstens 4 C-Atome aufweisen, besonders das Dimethylformamid, verwendet (DE-AS 11 78 052; Helv. Chim. Acta. 42, 1653 bis 1658 [1959]). Es ist jedoch inzwischen bekannt geworden, daß aus Dimethylformamid und Thionylchlorid oder Phosgen, die häufig für den Austausch einer OH-Gruppe gegen Chlor eingesetzt werden, N,N-Dialkylcarbamoylchloride entstehen (Isw. Akad. Nauk USSR, Chem. Ser. 1971 [3], 513–519; zitiert nach C.A. 75, 48 340 m). Diese erwiesen sich im Tierversuch als krebserregend (J. Nat. Cancer Inst. 48 [5], 1539–1541 (1972); zitiert nach C.A. 77, 97 540 b). Die N,N-Dialkylformamide mit niederen Alkylresten, besonders das Dimethylformamid, sind daher aus arbeitshygienischen Gründen bedenklich, so daß ihr Ersatz erforderlich erscheint.

Die erfindungsgemäßen N-alkylierten Formamide der Formel (I) gehen bei ihrer Verwendung nicht oder nur in vernachlässigbarem Maße in die Umwelt über, so daß ihre Verwendung arbeitshygienische Vorteile bringt. Überraschenderweise erweisen sie sich als katalytisch voll wirksam, obwohl in der zitierten DE-AS 11 78 052 die katalytisch wirksamen Alkylformamide auf solche mit kurzkettigen Alkylresten bis zu 4 C-Atomen beschränkt sind.

Als Verfahren des Austauschs von Hydroxylgruppen in organischen Verbindungen gegen Chlor oder Brom, der durch die erfindungsgemäßen N-alkylierten Formamide beschleunigt werden kann, sei beispielsweise die Herstellung von N-Heterocyclen, die in $\alpha$-Stellung zum Heterostickstoff mindestens 1 Halogenatom haben, aus den entsprechenden N-Heterocyclen, die in $\alpha$-Stellung mindestens eine Hydroxylgruppe tragen und einem anorganischen Säurehalogenid genannt. Insbesondere sei der genannte Austausch an aromatischen N-Heterocyclen genannt. Diese Reaktion kann in der folgenden allgemeinen Reaktionsgleichung dargestellt werden:

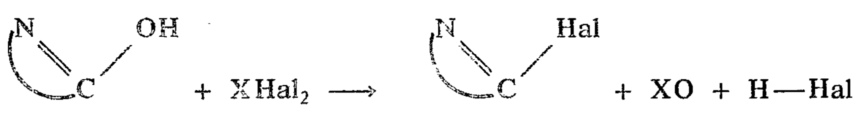

wobei in Gegenwart der erfindungsgemäßen Stoffe der Formel (I) gearbeitet wird und wobei

Hal für Chlor- oder Brom, bevorzugt Chlor, und

X für CO, SO oder $SO_2$ steht und

wobei die ringförmige Verbindungslinie zwischen N und C den heterocyclischen Ring bedeutet, der gegebenenfalls auch an der zweiten $\alpha$-Stellung zum N-Atom eine austauschbare Hydroxylgruppe enthält.

Als weiteres Beispiel für den Austausch einer Hydroxylgruppe in organischen Verbindungen gegen Chlor oder Brom sei die Herstellung von organischen Säurechloriden oder -bromiden, wie Carbonsäurechloriden, Carbonsäurebromiden, Sulfonsäurechloriden oder Sulfonsäurebromiden, aus den Säuren oder ihren Salzen und einem anorganischen Säurechlorid oder Säurebromid genannt. Diese Reaktionen können beispielsweise durch die folgende allgemeine Reaktionsgleichung beschrieben werden:

$$R-\overset{\overset{O}{\|}}{Y}-OH + XHal_2 \longrightarrow R-\overset{\overset{O}{\|}}{Y}-Hal + XO + H-Hal$$

wobei die erfindungsgemäßen Stoffe der Formel (I) als Beschleuniger eingesetzt werden und wobei

X und Hal die obengenannte Bedeutung haben,
R für Alkyl, Cycloalkyl, Aralkyl, Aryl oder Heteroaryl steht, wobei R weiterhin durch Alkyl, Cycloalkyl, Aryl, Heteroaryl, Halogen, Alkoxy, Aryloxy, Nitro, Cyano oder durch eine oder mehrere Carbonsäure- oder Sulfonsäuregruppen, die gegebenenfalls ebenfalls in die Säurehalogenidgruppen umgewandelt werden, substituiert sein kann, und
Y für ein C-Atom oder die SO-Gruppe steht.

Die obengenannte Umsetzung von phenolischen Verbindungen mit Phosgen zu Arylchlorameisensäureestern, die durch die erfindungsgemäßen Stoffe der Formel (I) ebenfalls beschleunigt werden kann, sei beispielsweise durch die folgende Reaktionsgleichung charakterisiert:

$$Ar-OH + COCl_2 \longrightarrow Ar-O-CO-Cl + HCl$$

wobei

Ar für einen Arylrest, beispielsweise Phenyl, Naphthyl oder Anthryl steht, der gegebenenfalls weitere Substituenten, wie für R offenbart, tragen kann.

Die Erfindung betrifft daher weiterhin die Verwendung der N-alkylierten Formamide der Formel (I) zur Beschleunigung des Austauschs von Hydroxylgruppen in organischen Verbindungen gegen Chlor oder Brom, insbesondere zur Beschleunigung des Austauschs von $\alpha$-ständigen Hydoxylgruppen in N-Heterocyclen und den in organischen Säuren enthaltenen OH-Gruppen unter Ausbildung der Säurehalogenidgruppen. Die Erfindung betrifft ebenso die Verwendung der N-alkylierten Formamide der Formel (I) zur Beschleunigung der Umsetzung von phenolischen Verbindungen mit Phosgen zu Arylchlorameisensäureestern.

Selbstverständlich können außer den reinen Stoffen der Formel (I) auch Gemische solcher Stoffe erfindungsgemäß eingesetzt werden. Die vorteilhaften Eigenschaften in bezug auf die volle katalytische Wirksamkeit und die arbeitshygienischen Eigenschaften der reinen Stoffe bleiben bei solchen Gemischen vollständig erhalten.

## Beispiel 1

### Herstellung von N-Methyl-stearylformamid

In einem 2-Ltr.-Dreihalskolben mit Rührer, Innenthermometer und Wasserabscheider werden 500 ml Toluol vorgelegt. Bei Raumtemperatur werden 566 g N-Methylstearylamin (99%ig) und anschließend 97 g Ameisensäure (99%ig) zugegeben. Dann wird so lange zum Sieden erhitzt, bis sich kein Wasser mehr abscheidet. Das Produktgemisch wird am Rotationsverdampfer bei 30 mbar und einer Badtemperatur bis 80°C von Toluol befreit.

Es werden 630 g N-Methylstearylformamid mit einem gaschromatographisch bestimmten Gehalt von 97% als Destillationsrückstand gewonnen.

Fp.: 34°C
Kp.: 185°C/1 mbar unter Zersetzung
Ausbeute: 99% der theoretischen Ausbeute.

Beispiel 2

227 g 2,3-Dihydroxychinoxalin-6-carbonsäure mit einem Gehalt von 91%werden in einem 1-Ltr.-Dreihalskolben, der mit Rührer, Innenthermometer und Rückflußkühler mit aufgesetztem Blasenzähler und Gasableitung versehen ist, vorgelegt. Dann werden bei Raumtemperatur 785 g Thionylchlorid und 18,6 g N-Methyl-stearylformamid zugegeben. Der Ansatz wird zum Sieden erhitzt, wobei eine heftige Gasentwicklung einsetzt. Die Siedetemperatur steigt während der Reaktion von etwa 55°C auf °C. Nach etwa 5 Stunden geht die Suspension in eine Lösung über, und nach weiteren 1–2 Stunden ist die Reaktion beendet, was am Ende der Gasentwicklung zu erkennen ist. Überschüssiges Thionylchlorid wird zunächst bei Normaldruck, dann bei 20 mbar bis zu einer Sumpftemperatur von 120°C abdestilliert. Es werden 130 g Thionylchlorid wiedergewonnen.

Der flüssige Rückstand wird auf ein Blech gegossen und nach dem Erkalten im Mörser pulverisiert. Es werden 276 g 2,3-Dichlorchinoxalin-6-carbonsäurechlorid isoliert. Das Produkt hat einen durch Sublimation bestimmten Gehalt von 94%. Die Ausbeute beträgt 99% der theoretischen Ausbeute.

Gehalt an N-Methyl-N-stearyl-carbamoylchlorid:

a) im Gasraum über dem heißen Produkt nicht nachweisbar
b) im zurückgewonnenen Thionylchlorid nicht nachweisbar.

Beispiel 3

In einem Vierhalskolben mit Rührer, Innenthermometer, Gaseinleitrohr und Rückflußkühler, dessen oberes Ende über zwei Waschflaschen mit einem Phosgenvernichtungsturm verbunden ist, werden 283,5 g (3,0 Mol) Chloressigsäure und 4,67 g (0,015 Mol) N-Methyl-stearylformamid bei 90°C vorgelegt.

Innerhalb von 7 Stunden werden durch das Einleitrohr 400 g Phosgen unter die Oberfläche des Ausgangsmaterials eingeleitet, bis die Reaktion beendet ist (erkennbar am Nachlassen der Gasentwicklung und am Absinken der Temperatur). Es wird noch 1 Std. bei der Reaktionstemperatur nachgerührt. Dann wird 1 Std. lang überschüssiges Phosgen bei 90°C mit Stickstoff ausgeblasen.

Das Produkt wird bei 105°C Kopftemperatur destilliert.

Hauptlauf: 298 g Chloracetylchlorid (88% der theoretischen Ausbeute).

Ein Vorlauf von 5 g kann dem Folgeansatz zugeführt werden. Das Produkt enthält weniger als die Nachweisgrenze von 1 ppm N-Methyl-stearyl-carbamoylchlorid in Chloracetylchlorid.

Beispiel 4

386,7 g (3,0 Mol) Dichloressigsäure und 4,67 g (0,015 Mol) N-Methyl-stearylformamid werden analog Beispiel 3 innerhalb von 10,5 Stunden mit 500 g Phosgen umgesetzt und aufgearbeitet.

Kopftemperatur bei der Destillation: 108–109°C.

Hauptlauf: 375 g Dichloracetylchlorid (85% der theoretischen Ausbeute).

Ein Vorlauf von 13 g kann dem Folgeansatz zugeführt werden. Das Produkt enthält weniger als die Nachweisgrenze von 1 ppm N-Methyl-stearyl-carbamoylchlorid in Dichloracetylchlorid.

Beispiel 5

238 g (1,75 Mol) o-Methylbenzoesäure und 2,75 g (0,009 Mol) N-Methylstearylformamid werden analog Beispiel 3 innerhalb 4 Std. mit 215 g Phosgen bei 100–105°C umgesetzt und aufgearbeitet.

Die Destillation erfolgt bei 20 mbar und einer Kopftemperatur von 102°C.

Hauptlauf: 262 g o-Methylbenzoylchlorid (97% der theoretischen Ausbeute).

Ein Vorlauf von 5 g kann dem Folgeansatz zugeführt werden. Das Produkt enthält weniger als die Nachweisgrenze von 100 ppm N-Methyl-stearyl-carbamoylchlorid in o-Methylbenzoylchlorid.

Beispiel 6

190 g (1,22 Mol) o-Methoxybenzoesäure und 3,8 g (0,012 Mol) N-Methyl-stearylformamid werden analog Beispiel 3 innerhalb 3,5 Std. mit 200 g Phosgen bei 100–105°C umgesetzt und aufgearbeitet.

Die Destillation erfolgt bei 20 mbar und einer Kopftemperatur von 142°C.

Hauptlauf: 199 g o-Methoxybenzoylchlorid (93% der theoretischen Ausbeute).

Ein Vorlauf fällt nicht an.

Das Produkt enthält weniger als die Nachweisgrenze von 50 ppm N-Methylstearylcarbamoylchlorid in o-Methoxybenzoylchlorid.

### Beispiel 7

188 g (1,2 Mol) m-Chlorbenzoesäure und 3,73 g (0,012 Mol) N-Methylstearylformamid werden analog Beispiel 3 innerhalb 5,5 Stunden mit 220 g Phosgen bei 110°C umgesetzt und aufgearbeitet.
Die Destillation erfolgt bei 20 mbar und einer Kopftemperatur von 110°C.
Hauptlauf: 201 g m-Chlorbenzoylchlorid (96% der theoretischen Ausbeute).
Ein Vorlauf von 4 g kann dem Folgeansatz zugeführt werden. Das Produkt enthält 35 ppm N-Methylstearylcarbamoylchlorid.

### Beispiel 8

188 g (1,2 Mol) p-Chlorbenzoesäure und 3,73 g (0,012 Mol) N-Methylstearylformamid werden analog Beispiel 3 innerhalb von 3,5 Std. mit 220 g Phosgen bei 110°C umgesetzt und aufgearbeitet.
Die Destillation erfolgt bei 20 mbar und einer Kopftemperatur von 109°C.
Hauptlauf: 196,5 g p-Chlorbenzoylchlorid (94% der theoretischen Ausbeute).
Ein Vorlauf von 4 g kann dem Folgeansatz zugeführt werden. Das Produkt enthält 18 ppm N-Methylstearylcarbamoylchlorid.

### Beispiel 9

Ausführung wie Beispiel 8 mit folgenden Mengen und Ergebnissen:

| | |
|---|---|
| 476 g (3,5 Mol) | m-Methylbenzoesäure |
| 5,5 g (0,016 Mol) | N-Methylstearylformamid |
| 430 g | Phosgen |

bei 104—110°C in 5½ Std.; Dest. 90—92°/16 mbar.

| | | |
|---|---|---|
| Ausbeute: | Hauptlauf | 528 g (97,6% der theoretischen Ausbeute), |
| | Vorlauf | <2 g. |

Das Produkt enthält unter 100 ppm Methyl-stearyl-carbamoylchlorid, weniger als die Nachweisgrenze in m-Methylbenzoylchlorid.

### Beispiel 10

In einem Vierhalskolben mit Rührer, Innenthermometer, Gaseinleitrohr und mit Trockeneis beschicktem Rückflußkühler, der mit einem Phosgenvernichtungsturm verbunden ist, werden 500 g Phenol und 33,1 g N-Methylstearylformamid vorgelegt.
Dann werden bei 120—125°C innerhalb 12 Std. 550 g Phosgen eingeleitet. Überschüssiges Phosgen wird abgezogen. Das Produkt wird im Wasserstrahlvakuum destilliert.

| | |
|---|---|
| Hauptfraktion 80—82°C/20 mbar | 727,5 g (99,7%ig), |
| Nachlauf | 108,2 g (34,4%ig). |

Ausbeute Chlorameisensäurephenylester: 91,2% der theoretischen Ausbeute.
Das Produkt enthält 4 ppm N-Methylstearylcarbamoylchlorid.

### Beispiel 11

In einer Apparatur, wie in Beispiel 10 beschrieben, werden 136,2 g (1 Mol) o-Methylbenzoesäure und 3,1 g (0,01 Mol) N-Methylstearylformamid vorgelegt. Bei 100°C werden innerhalb 4 Std. 130 g Phosgen eingeleitet. Nach Abziehen des überschüssigen Phosgens wird das Produkt an der Ölpumpe destilliert (2—3 mbar, 66°C).
Hauptlauf: 149,5 g o-Methylbenzoylchlorid (97% der theoretischen Ausbeute).
Das Produkt enthält weniger als die Nachweisgrenze von 100 ppm N-Methylstearylcarbamoylchlorid in o-Methylbenzoylchlorid.

Beispiel 12

In einem Dreihalskolben mit Rührer, Innenthermometer und Rückflußkühler werden 166,2 g (1 Mol) Isophthalsäure und 297,5 g (2,5 Mol) Thionylchlorid mit 1 g (0,003 Mol) N-Methylstearylformamid 4 Std. unter Rückfluß erhitzt. Überschüssiges Thionylchlorid wird bei 30 mbar bis zu einer Sumpftemperatur von 100°C abdestilliert. Als Rückstand verbleiben 202,4 g Isophthaloylchlorid mit einem Gehalt von 98%.
Ausbeute: 97,7% der theoretischen Ausbeute.

Beispiel 13

(Vergleich zu Beispiel 6)

Ausführung wie Beispiel 6 mit folgenden Mengen und Ergebnissen:

| | |
|---|---|
| 190 g (1,22 Mol) | o-Methoxybenzoesäure |
| 0,74 g (0,01 Mol) | Dimethylformamid |
| 200 g | Phosgen |

Ausbeute: Hauptlauf 206 g.
Produkt enthält etwa 400 ppm Dimethylcarbamoylchlorid.

Beispiel 14

(Vergleich zu Beispiel 5 und 11)

Ausführung wie Beispiel 3 mit folgenden Mengen und Ergebnissen:

| | |
|---|---|
| 476 g (3,5 Mol) | m-Methylbenzoesäure |
| 2,56 g (1,0 Mol-%) | Dimethylformamid |
| 430 g | Phosgen |

bei 100—110°C in 5 Std., Dest. 90—92°/16 mbar.

| | | |
|---|---|---|
| Ausbeute: | Hauptlauf | 534 g, |
| | Vorlauf | 3 g. |

Im Hauptlauf lassen sich 1100 ppm, im Vorlauf 1500 ppm Dimethylcarbamoylchlorid nachweisen.

Beispiel 15

In einem 500-ml-Dreihalskolben mit Rührer, Innenthermometer und Wasserabscheider werden 300 ml Toluol vorgelegt. Bei Raumtemperatur werden 70,5 g N,N',N"-Trimethyl-diethylentriamin (89%ig) und anschließend 67 g Ameisensäure (99%ig) zugegeben.
Reaktion und Abdestillieren des Toluols erfolgen wie in Beispiel 1.
Der Eindampfrückstand wird in Vakuum destilliert.
Als Hauptfraktion erhält man 88 g $\alpha$, $\omega$-Bisformyl-N,N',N"-trimethyl-diethylentriamin mit einem Gehalt von 97%.
Kp. 182—185°C/1,3 mbar.
Ausbeute: 98% der theoretischen Ausbeute.

Beispiel 16

113,2 g 2,3-Dihydroxychinoxalin-6-carbonsäure mit einem Gehalt von 91% werden in einem 0,5-l-Dreihalskolben mit 393 g Thionylchlorid in Gegenwart von 4,6 g $\alpha$, $\omega$-Bisformyl-N,N',N"-trimethyl-diethylentriamin analog Beispiel 2 umgesetzt und aufgearbeitet.
Es werden 99 g Thionylchlorid wiedergewonnen.
135 g 2,3-Dichlorchinoxalin-6-carbonsäurechlorid mit einem durch Sublimation bestimmten Gehalt von 88% werden isoliert. Die Ausbeute beträgt 91% der theoretischen Ausbeute.
Eine dem Katalysator entsprechende Carbamoylverbindung konnte nicht nachgewiesen werden.

Beispiel 17—19

Analog Beispiel 1 werden die folgenden Formamide hergestellt:

| Beispiel | Formamid | Kp | Ausbeute |
|---|---|---|---|
| 1 a | N-Methyltetradecylformamid | 130°C/0,3 mbar | 98% |
| 1 b | N-Methylhexadecylformamid | 165°C/0,4 mbar | 99% |
| 1 c | N-Ethylstearylformamid | 190°C/0,4 mbar | 99% |

Beispiel 20

In einem 2-Ltr.-Kolben mit Rührer, Innenthermometer, Vigreux-Kolonne und Destillationseinrichtung werden 1013 g N-Methylstearylamin bei 105°C vorgelegt. 173,6 g Ameisensäure werden während einer Stunde bei 105°C zugetropft. Nach einer weiteren Stunde bei 105°C wird dann innerhalb 1 ½ Stunden auf 120°C geheizt, wobei ca. 41 g Wasser destillieren. Unter allmählichem Anlegen eines Vakuums von 8 mbar werden weitere 15 ml Wasser abdestilliert. Es wird noch eine Stunde bei 120°C/8 mbar nachgerührt. Als Rückstand verbleiben
1109 g Methylstearlyformamid (97,2%ig)
Ausbeute: 99% der theoretischen Ausbeute
Fp.: 33—34°C.

Beispiel 21

In einer Rührwerksapparatur mit Gaseinleitungsrohr, Thermometer und Rückflußkühler, über eine Gassicherheitsflasche mit einem Phosgenvernichtungsturm verbunden, werden

476 g m-Methylbenzoesäure (3,50 Mol) und
3,5 g $\alpha$, $\omega$-Bis-formyl-N,N',N''-trimethyl-diethylentriamin mit
565 g Phosgen in 7 Std. bei 104—108°C umgesetzt.

Das Rohprodukt wird durch einstündiges Verrühren bei 110°C im Stickstoffstrom von Phosgen befreit. Destillation bei 102—104°C/27 mbar liefert 528 g farbloses m-Methylbenzoylchlorid (97,6% der theoretischen Ausbeute).
Gehalt über 99,6%.
Das dem Katalysator entsprechende Carbamoylchlorid ist nicht nachweisbar (Erfassungsgrenze 20 ppm).

Beispiel 22

In o.g. Apparatur werden

190 g o-Methoxybenzoesäure (1,24 Mol) und
2,5 g $\alpha$, $\omega$-Bis-formyl-N,N',N''-trimethyl-diethylentriamin mit
230 g Phosgen in 4 Std. bei 100—108°C umgesetzt.

Nach Phosgenentfernung und Destillation entstehen 198,6 g farbloses o-Methoxybenzoylchlorid (93% der theoretischen Ausbeute).
Gehalt über 99,6%.
Das dem Katalysator entsprechende Carbamoylchlorid ist nicht nachweisbar (Erfassungsgrenze 100 ppm).

**Patentansprüche**

1. N-alkylierte Formamide der Formel

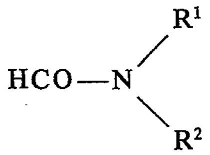

in der

$R^1$ niederes Alkyl und
$R^2$ geradkettiges Alkyl mit 14–30 C-Atomen oder die Gruppe

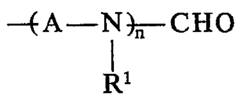

bedeutet, wobei

A geradkettiges oder verzweigtes Alkandiyl mit 2 bis 8 C-Atomen,
$R^1$ niederes Alkyl und
n eine ganze Zahl von 2 bis 6

bedeutet.

2. N-Alkylmethylformamide der Formel

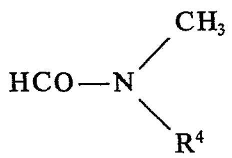

in der

$R^4$ geradkettiges $C_{16}$–$C_{22}$-Alkyl bedeutet.

3. N-alkylierte Formamide der Formel

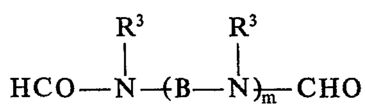

in der

$R^3$ $C_1$–$C_4$-Alkyl,
B geradkettiges oder verzweigtes $C_2$–$C_4$-Alkandiyl und
m eine ganze Zahl von 2 bis 4 bedeuten.

4. N-alkylierte Formamide der Formel

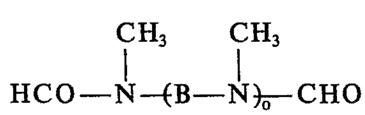

in der

o die Zahl 2 oder 3 bedeutet und
B für geradkettiges oder verzweigtes $C_2$–$C_4$-Alkandiyl steht.

5. Gemische aus 2 oder mehr Stoffen nach Anspruch 1.

6. Verfahren zur Herstellung von N-alkylierten Formamiden der Formel

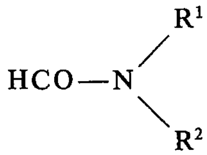

in der

$R^1$ niederes Alkyl und
$R^2$ geradkettiges Alkyl mit 14—30 C-Atomen oder die Gruppe

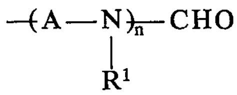

bedeutet, wobei

A geradkettiges oder verzweigtes Alkandiyl mit 2 bis 8 C-Atomen,
$R^1$ niederes Alkyl und
n eine ganze Zahl von 2 bis 6 bedeutet,

dadurch gekennzeichnet, daß man N-alkylierte Amine der Formel

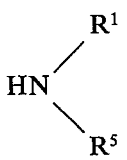

in der

$R^1$ die genannte Bedeutung hat und
$R^5$ geradkettiges Alkyl mit 14—30 C-Atomen oder die Gruppe

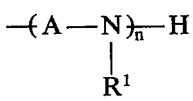

bedeutet, wobei

A geradkettiges oder verzweigtes Alkandiyl mit 2 bis 8 C-Atomen,
$R^1$ niederes Alkyl und
n eine ganze Zahl von 2 bis 6 bedeutet,

mit Ameisensäure bei erhöhter Temperatur, gegebenenfalls in Gegenwart eines inerten Lösungsmittels, umsetzt.

7. Verwendung von N-alkylierten Formamiden der Formel

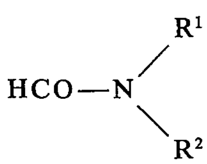

in der

$R^1$ niederes Alkyl und
$R^2$ geradkettiges Alkyl mit 14—30 C-Atomen oder die Gruppe

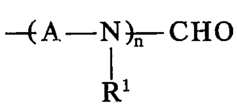

bedeutet, wobei

- A geradkettiges oder verzweigtes Alkandiyl mit 2 bis 8 C-Atomen,
- $R^1$ niederes Alkyl und
- n eine ganze Zahl von 2 bis 6

bedeutet,
zur Beschleunigung des Austausches von Hydroxylgruppen in organischen Verbindungen gegen Chlor oder Brom.

8. Verwendung nach Anspruch 7, dadurch gekennzeichnet, daß die N-alkylierten Formamide nach den Ansprüchen 1 bis 5 bei der Herstellung von N-Heterocyclen, die in $\alpha$-Stellung zum Heterostickstoff mindestens ein Halogenatom haben, aus dem entsprechenden N-Heterocyclen, die in $\alpha$-Stellung mindestens eine OH-Gruppe tragen, und einem anorganischen Säurehalogenid eingesetzt werden.

9. Verwendung nach Anspruch 7, dadurch gekennzeichnet, daß die N-alkylierten Formamide mit 2 bis 8 C-Atomen nach den Ansprüchen 1 bis 5 bei der Herstellung von organischen Säurechloriden oder -bromiden aus den Säuren oder ihren Salzen und einem anorganischen Säurechlorid eingesetzt werden.

10. Verwendung von N-alkylierten Formamiden der Formel

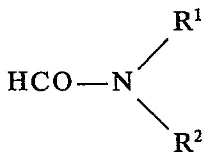

in der

- $R^1$ niederes Alkyl und
- $R^2$ geradkettiges Alkyl mit 14—30 C-Atomen oder die Gruppe

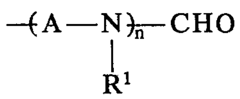

bedeutet, wobei

- A geradkettiges oder verzweigtes Alkandiyl mit 2 bis 8 C-Atomen,
- $R^1$ niederes Alkyl und
- n eine ganze Zahl von 2 bis 6 bedeutet,

zur Beschleunigung der Umsetzung von phenolischen Verbindungen mit Phosgen zu Arylchlorameisensäureestern.

## Claims

1. N-Alkylated formamides of the formula

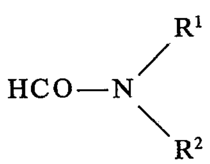

in which

- $R^1$ denotes lower alkyl and
- $R^2$ denotes straight-chain alkyl with 14—30 C atoms or the group

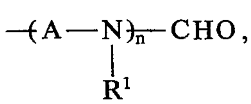

wherein

A denotes straight-chain or branched alkanediyl with 2 to 8 C atoms,
$R^1$ denotes lower alkyl and
n denotes an integer from 2 to 6.

2. N-Alkylmethylformamides of the formula

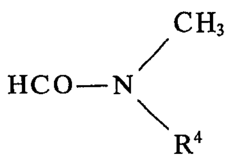

in which

$R^4$ denotes straight-chain $C_{16}$—$C_{22}$-alkyl.

3. N-Alkylated formamides of the formula

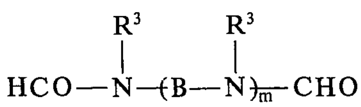

in which

$R^3$ denotes $C_1$—$C_4$-alkyl,
B denotes straight-chain or branched $C_2$—$C_4$-alkanediyl and
m denotes an integer from 2 to 4.

4. N-Alkylated formamides of the formula

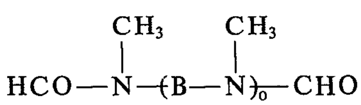

in which

o denotes the number 2 or 3 and
B represents straight-chain or branched $C_2$—$C_4$-alkanediyl.

5. Mixtures or 2 or more substances according to Claim 1.
6. Process for the preparation of N-alkylated formamides of the formula

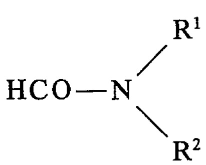

in which

$R^1$ denotes lower alkyl and
$R^2$ denotes straight-alkyl with 14—30 C atoms or the group

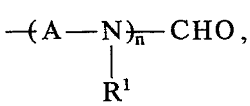

wherein

A denotes straight-chain or branched alkanediyl with 2 to 8 C atoms,
$R^1$ denotes lower alkyl and
n denotes an integer from 2 to 6,

characterised in that N-alkylated amines of the formula

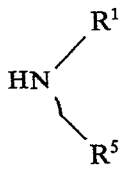

in which

$R^1$ has the indicated meaning and
$R^5$ denotes straight-chain alkyl with 14—30 C atoms or the group

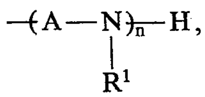

wherein

A denotes straight-chain or branches alkanediyl with 2 to 8 C atoms,
$R^1$ denotes lower alkyl and
n denotes an integer from 2 to 6,

are reacted with formic acid at elevated temperature, if appropriate in the presence of an inert solvent.

7. Use of N-alkylated formamides of the formula

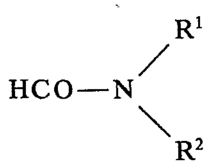

in which

$R^1$ denotes lower alkyl and
$R^2$ denotes straight-chain with 14—30 C atoms or the group

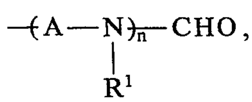

wherein

A denotes straight-chain or branched alkanediyl with 2 to 8 C atoms,
$R^1$ denotes lower alkyl and
n denotes an integer from 2 to 6,

to accelerate the replacement of hydroxyl groups in organic compounds by chlorine or bromine.

8. Use according to Claim 7, characterised in that the N-alkylated formamides according to Claims 1 to 5 are employed in the preparation of N-heterocyclic compounds which have at least one halogen atom in the $\alpha$-position relative to the hetero-nitrogen from the corresponding N-heterocyclic compounds which carry at least one OH group in the $\alpha$-position and an inorganic acid halide.

9. Use according to Claim 7, characterised in that the N-alkylated formamides with 2 to 8 C atoms according to Claims 1 to 5 are employed in the preparation of organic acid chlorides or bromides from the acids or their salts and an inorganic acid chloride or acid bromide.

10. Use of N-alkylated formamides of the formula

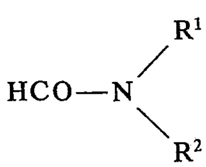

in which

$R^1$ denotes lower alkyl and
$R^2$ denotes straight-chain alkyl with 14—30 C atoms or the group

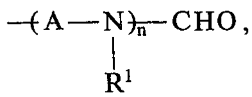

wherein

A denotes straight-chain or branched alkanediyl with 2 to 8 C atoms,
$R^1$ denotes lower alkyl and
n denotes an integer from 2 to 6,

to accelerate the reaction of phenolic compounds with phosgene to give aryl chloroformates.

**Revendications**

1. Formamides N-alkylés de formule

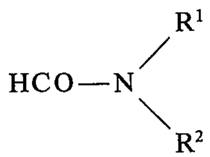

dans laquelle

$R^1$ est un reste alkyle inférieur et
$R^2$ est un reste alkyle à chaîne droite ayant 14 à 30 atomes de carbone ou le groupe

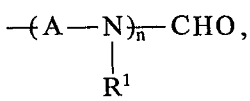

où

A est un reste alcanediyle à chaîne droite ou ramifié ayant 2 à 8 atomes de carbone,
$R^1$ est un reste alkyle inférieur et
n est un nombre entier de 2 à 6.

2. N-alkylméthylformamides de formule

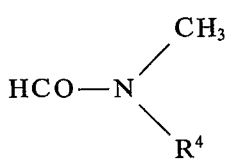

dans laquelle

$R^4$ est un reste alkyle en $C_{16}$ à $C_{22}$ à chaîne droite.

3. Formamides N-alkylés de formule

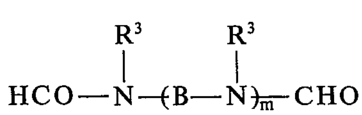

dans laquelle

$R^3$ est un reste alkyle en $C_1$ à $C_4$,
B est un reste alcanediyle en $C_2$ à $C_4$ à chaîne droite ou ramifié et

m est un nombre entier de 2 à 4.

4. formamide N-alkylés de formule

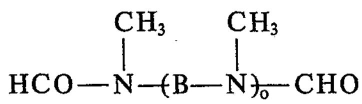

dans laquelle

o désigne le nombre 2 ou 3 et
B est un reste alcanediyle en $C_2$ à $C_4$ à chaîne droite ou ramifié.

5. Des mélanges de 2 ou plus de deux substances suivant la revendication 1.

6. Procédé de production de formamides N-alkyles de formule

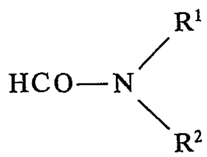

dans laquelle

$R^1$ est un reste alkyle inférieur et
$R^2$ est un reste alkyle à chaîne droite ayant 14 à 30 atomes de carbone ou le groupe

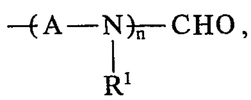

dans lequel

A est un reste alcanediyle à chaîne droite ou ramifié ayant 2 à 8 atomes de carbone,
$R^1$ est un reste alkyle inférieur et
n est un nombre entier de 2 à 6,

caractérisé en ce qu'on fait réagir des amines N-alkylées de formule

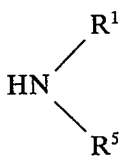

dans laquelle

$R^1$ a la définition mentionée et
$R^5$ est un reste alkyle à chaîne droite ayant 14 à 30 atomes de carbone ou le groupe

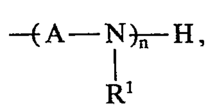

dans lequel

A est un reste alcanediyle à chaîne droite ou ramifié 2 à 8 atomes de carbone,
$R^1$ est un reste alkyle inférieur et
n est un nombre entier de 2 à 6,

avec l'acide formique à température élevée, éventuellement en présence d'un solvant inerte.

7. Utilisation de formamides N-alkylés de formule

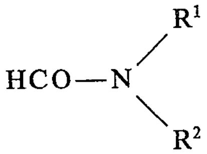

dans laquelle

$R^1$ est un reste alkyle inférieur et
$R^2$ est un reste à chaîne droite ayant 14 à 30 atomes de carbone ou le groupe

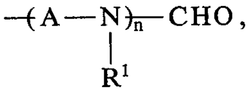

dans lequel

A est un reste alcanediyle à chaîne droite ou ramifié ayant 2 à 8 atomes de carbone,
$R^1$ est un reste alkyle inférieur et
n est un nombre entier de 2 à 6,

pour l'accélération de l'échange de groupes hydroxyle dans des composés organiques contre du chlore ou du brome.

8. Utilisation suivant la revendication 7, caractérisée en ce que les formamides N-alkylés selon les revendications 1 à 5 sont utilisés dans la production de N-hétérocycles, qui portent en position $\alpha$ par rapport à l'hétéro-atome d'azote au moins un atome d'halogène, à partir des N-hétérocycles correspondants qui portent au moins un groupe OH en position $\alpha$ et d'un halogénure d'acide inorganique.

9. Utilisation suivant la revendication 7, caractérisée en ce que les formamides N-alkylés ayant 2 à 8 atomes de carbone selon les revendications 1 à 5 sont utilisés dans la production de chlorures ou bromures d'acides organiques à partir des acides ou de leurs sels et d'un chlorure ou bromure d'acide inorganique.

10. Utilisation de formamides N-alkylés de formule

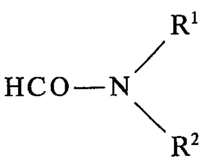

dans laquelle

$R^1$ est un reste alkyle inférieur et
$R^2$ est un reste alkyle à chaîne droite ayant 14 à 30 atomes de carbone ou le groupe

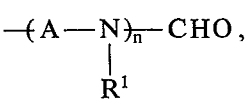

dans lequel

A est un reste alcanediyle à chaîne droite ou ramifié ayant 2 à 8 atomes de carbone,
$R^1$ est un reste alkyle inférieur et
n est un nombre entier de 2 à 6,

pour l'accélération de la formation d'esters d'acides arylchloroformiques par réaction de composés phénoliques avec le phosgène.